(19) Europäisches Patentamt
European Patent Office
Office européen des brevets

(11) **EP 3 653 119 A1**

(12) **EUROPEAN PATENT APPLICATION**

(43) Date of publication:
**20.05.2020 Bulletin 2020/21**

(51) Int Cl.:
**A61B 5/08** *(2006.01)*          **A61B 5/1455** *(2006.01)*
**A61M 16/12** *(2006.01)*

(21) Application number: **19206524.1**

(22) Date of filing: **13.03.2014**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.03.2013  US 201361779301 P**

(62) Document number(s) of the earlier application(s) in
accordance with Art. 76 EPC:
**14722023.0 / 2 967 434**

(71) Applicant: **Ino Therapeutics LLC
Hampton, New Jersey 08827-9001 (US)**

(72) Inventors:
• **FLANAGAN, Craig
Belmar, NJ New Jersey 07719 (US)**

• **NEWMAN, David
Lebanon, NJ New Jersey 08833 (US)**
• **ACKER, Jaron
Madison, WI Wisconsin 53703 (US)**
• **TOLMIE, Craig R.
Stoughton, WI Wisconsin 53589 (US)**

(74) Representative: **Jones, Nicholas Andrew
Withers & Rogers LLP
4 More London Riverside
London, SE1 2AU (GB)**

Remarks:
This application was filed on 31-10-2019 as a
divisional application to the application mentioned
under INID code 62.

(54) **DEVICES FOR MONITORING OXYGENATION DURING TREATMENT WITH DELIVERY OF NITRIC OXIDE**

(57)    The present invention provides devices and methods for calculating and monitoring oxygenation parameters during treatment with delivery of nitric oxide. The devices and methods of the present invention can calculate the oxygenation index based on measurements of mean airway pressure, saturation ofp oxygen and fraction of inspired oxygen derived from components of the present invention. Also described is a nitric oxide delivery device that incorporates a proximal pressure transducer.

Figure 1

**Description**

FIELD OF THE INVENTION

The present invention relates to devices and methods for calculating, monitoring and trending oxygenation parameters during treatment with inhaled nitric oxide while on mechanical ventilation or non-invasive support.

BACKGROUND

The safety and effectiveness of inhaled nitric oxide (NO) has been established in patients receiving therapies for hypoxic respiratory failure, including vasodilators, intravenous fluids, bicarbonate therapy, and mechanical ventilation. Methods for safe and effective administration of NO by inhalation are well known in the art. NO for inhalation is available commercially. NO inhalation preferably is in accordance with established medical practice.

Inhaled nitric oxide (iNO) is a vasodilator indicated for treatment of hypoxic respiratory failure associated with clinical or echocardiographic evidence of pulmonary hypertension. In patients, iNO has been shown to improve oxygenation and reduce the need for extracorporeal membrane oxygenation (ECMO) therapy. NO binds to and activates cytosolic guanylate cyclase, thereby increasing intracellular levels of cyclic guanosine 3',5'-monophosphate (cGMP). This, in turn, relaxes vascular smooth muscle, leading to vasodilatation. Inhaled NO selectively dilates the pulmonary vasculature, with minimal systemic vasculature effect as a result of efficient hemoglobin scavenging. In acute lung injury (ALI) and acute respiratory distress syndrome (ARDS), increases in partial pressure of arterial oxygen ($PaO_2$) are believed to occur secondary to pulmonary vessel dilation in better-ventilated lung regions. As a result, pulmonary blood flow is redistributed away from lung regions with low ventilation/perfusion ratios toward regions with normal ratios.

Methemoglobinemia is a dose-dependent side effect of inhaled nitric oxide therapy. Elevation in methemoglobin is a known toxicity of inhaled nitric oxide (NO) therapy. Therefore, it can be desirable to monitor methemoglobin levels and oxygenation index during the administration of inhaled nitric oxide therapy.

Moreover, nitrogen dioxide ($NO_2$) rapidly forms in gas mixtures containing nitric oxide and oxygen. $NO_2$ formed in this way can cause airway inflammation and damage.

Various forms of oxygenation indicators have been used to track the progress or regression of the patient over time while on a ventilator. Examples include: Oxygenation Index (OI), Oxygen Saturation Index (OSI), $PaO_2/FiO_2$ ratio (P/F ratio) and Respiratory Severity Index (RSI). However, these oxygenation indicators can be burdensome to monitor with current methods. Therefore, a device and method for calculating and monitoring oxygenation indicators during treatment with delivery of nitric oxide is desired.

SUMMARY OF THE INVENTION

One aspect of the present invention pertains to a device for calculating and monitoring oxygenation during treatment with delivery of nitric oxide. In one or more embodiments of this aspect, the device comprises a first inlet to be placed in fluid communication with a therapeutic gas supply comprising nitric oxide or a nitric oxide-releasing agent; a second inlet to be placed in fluid communication with a flow of breathing gas; an outlet to be placed in fluid communication with the first inlet, the second inlet, and a patient; a proximal pressure transducer for determining mean airway pressure; a $FiO_2$ measurement means for measuring fraction of inspired oxygen ($FiO_2$); an oxygen measurement means for measuring one or more oxygen measurements selected from the group consisting of arterial oxygen saturation ($SaO_2$), peripheral oxygen saturation ($SpO_2$) and partial pressure of oxygen in arterial blood ($PaO_2$); a signal processor capable of calculating oxygenation parameter based upon a mean airway pressure measurement obtained from the proximal pressure transducer, an oxygen measurement obtained from the oxygen measurement means, and a $FiO_2$ measurement obtained from the $FiO_2$ measurement means; and a display. The oxygenation parameter may include one or more of oxygenation index or oxygen saturation index.

The signal processor may be a central processing unit. In one or more embodiments, the $FiO_2$ measurement means may comprise a ventilator or a $FiO_2$ sensor. In one or more embodiments, the oxygen measurement means may comprise a pulse oximeter and the oxygen measurement may comprise $SpO_2$. In one or more embodiments, the device may further comprise a continuous blood gas monitor to measure $PaO_2$ or a transcutaneous blood gas monitor with oxygen measurement comprised of $TcO_2$.

In one or more embodiments, the device may also include an alarm system operable by a signal from said signal processor indicative of a predetermined value of oxygenation index, oxygen saturation index, $SpO_2$, $SaO_2$, methemoglobin, or airway pressure.

In one or more embodiments, the device may further comprise a flow transducer to measure the flow of breathing gas and a control system in communication with the flow transducer. In one or more embodiments, the flow transducer may be integral to an injector module that combines the flows of breathing gas and therapeutic gas comprising nitric oxide or a nitric oxide-releasing agent.

In one or more embodiments, the device may further comprise one or more control valves to deliver a flow of the therapeutic gas comprising nitric oxide or a nitric oxide-releasing agent in an amount to provide a predetermined concentration of nitric oxide to a patient.

The signal processor may be configured to communicate with the $FiO_2$ measurement means that measures fraction of inspired oxygen ($FiO_2$) and an oxygen measurement means that measures one or more oxygen measurements selected from the group consisting of arterial oxygen saturation ($SaO_2$), peripheral oxygen saturation ($SpO_2$) and partial pressure of oxygen in arterial blood ($PaO_2$).

The display may show calculated values of methemoglobin, oxygenation index, oxygen saturation index, $SpO_2$, $SaO_2$, and airway pressure.

In one or more embodiments, the device may further comprise a transmitter to transmit calculated values of methemoglobin, oxygenation index, oxygen saturation index, $SpO_2$, $SaO_2$, and airway pressure to a remote information management system.

In one or more embodiments, the device may further comprise a purge valve.

Another aspect of the present invention pertains to a method of monitoring oxygenation index comprising the steps of: obtaining a mean airway pressure (MAP) measurement from a proximal pressure transducer; obtaining one or more oxygen measurements selected from the group consisting of arterial oxygen saturation ($SaO_2$), peripheral oxygen saturation ($SpO_2$), partial pressure of oxygen in arterial blood ($PaO_2$) and $TcO_2$ from an oxygen measurement means; obtaining a fraction of inspired oxygen ($FiO_2$) measurement from a $FiO_2$ measurement means; transmitting the MAP measurement, the oxygen measurement and the $FiO_2$ measurement to a signal processor; calculating an oxygenation parameter value via the signal processor and conveying the oxygenation parameter to an end user via a display. In one or more embodiments, the oxygenation parameter is calculated using the following equation:

$$OI = \frac{F_iO_2 * MAP}{PaO_2}$$

Depending on the oxygenation parameter, other oxygen measurements such as $SaO_2$, $SpO_2$ or $TcO_2$, may be used in place of $PaO_2$ in the above equation. The value may also be multiplied by 100 as is customary in practice.

In one or more embodiments, the method may further comprise administering a therapeutic gas comprising nitric oxide to a patient. In one or more embodiments, the method may further comprise comparing the oxygenation parameter value to a predetermined high value limit and emitting an alarm if the oxygenation parameter is above the high value limit.

BRIEF DESCRIPTION OF THE DRAWINGS

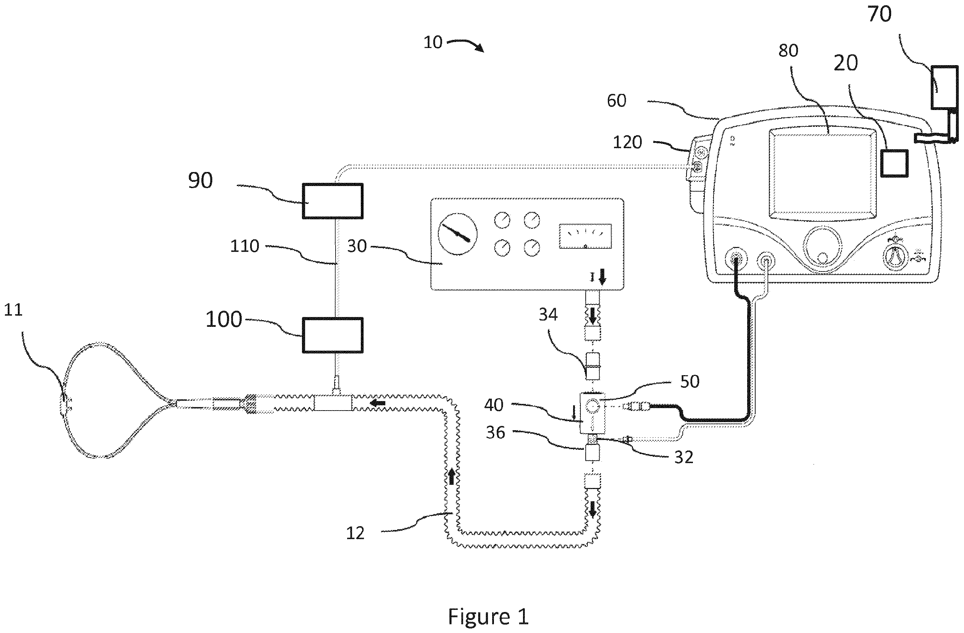

Figure 1 shows an embodiment of the device of the present invention.

DETAILED DESCRIPTION

Before describing several exemplary embodiments of the invention, it is to be understood that the invention is not limited to the details of construction or process steps set forth in the following description. The invention is capable of other embodiments and of being practiced or being carried out in various ways.

One aspect of the present invention relates to a device for determining the oxygenation parameter of an individual. The term "individual" is herein understood as a member selected from the group comprising humans, as well as, farm animals, domestic animals, pet animals and animals used for experiments such as monkeys, rats, rabbits, etc.

As described herein, oxygenation parameters are parameters that describe the relationship between a patient's venilator parameters and a patient's oxygen status. Such oxygenation parameters include, but are not limited to, oxygenation index (OI), oxygen saturation index (OSI), $PaO_2/FiO_2$ ratio (P/F ratio) and respiratory severity index (RSI). Ventilator parameters include, but are not limited to, $FiO_2$, MAP, peak airway pressure, CPAP, etc. The patient's oxygen status may be represented by several parameters including, but not limited to, $PaO_2$, $SaO_2$, $SpO_2$, $TcO_2$, etc.

For the ease of description, only one or several of these oxygenation parameters may be explicitly described below, but other parameters may be calculated, displayed and monitored using the appropriate equations.

For example, the oxygenation index of a patient may be calculated using the following equation:

$$OI = \frac{F_iO_2 * MAP}{PaO_2}$$

Similarly, the oxygen saturation index of a patient may be calculated using the following equation:

$$OSI = \frac{F_iO_2 * MAP}{SpO_2}$$

and the respiratory severity index may be calculated using the following equation:

$$RSI = F_iO_2 * MAP$$

As shown in Figure 1, one or more embodiments of the present invention relates to a device 10 for determining one or more respiratory parameters relating to an individual, comprising a central processing unit (CPU) 20 for determining said one or more respiratory parameters, a breathing gas delivery means 30, such as a ventilator, for detecting the level of oxygen ($FiO_2$, Mean Airway Pressure, etc.) in the gas flow passing into or out of the respiratory system of the individual, a proximal pressure transducer 100, a pulse oximeter 90 or other oxygen measurement means for detecting the level of oxygen ($SaO_2$, $SpO_2$, $PaO_2$) in the blood circulation of the individual and producing an output to the computer accordingly, the computer being adapted for calculating, retrieving and storing one or more measurements of oxygenation parameters. In one or more embodiments, the device may comprise a blood gas monitor to measure $PaO_2$. The term "respiratory parameters" is herein understood as parameters relating to oxygen transport from the lungs to the blood, such as parameters related to oxygenation index, abnormal ventilation, resistance to oxygen uptake from the lungs to the lung capillary blood, and parameters related to shunting of venous blood to the arterial blood stream. These respiratory parameters may be given as absolute values or relative values as compared to a set of standard values. The parameters may further be normalized or generalized to obtain parameters that are comparable to similar parameters measured for other individuals, at least for individuals of the same species.

Various embodiments of the present invention are directed to a device and method for calculating and monitoring oxygenation parameters during treatment with inhaled nitric oxide. The clinical aspect of oxygenation index for NO therapy is that OI and OSI provide useful information to a physician practitioner in aiding treatment decisions with respect to initiation or continuation of NO therapy, effectiveness of treatment and assessing patient progress.

Certain embodiments of the invention generally provide a device 10 for delivering a therapeutic gas comprising nitric oxide to a patient. The therapeutic gas comprises nitric oxide in a carrier gas such nitrogen. Suitable therapeutic gases can have varying concentrations of nitric oxide, ranging from 100 ppm to 1000 ppm.

As shown in FIG. 1, a source of the pharmaceutical gas is provided by means of a gas supply tank 70 containing the pharmaceutical gas generally in a carrier gas. When the pharmaceutical gas is NO, the carrier gas is conventionally nitrogen and the typical available concentrations range from 100 ppm to 1600 ppm.

Accordingly, from the supply tank, there is a tank pressure gauge and a regulator to bring the tank pressure down to the working pressure of the gas delivery system. The pharmaceutical gas enters the gas delivery system through an inlet that can provide a ready connection between that delivery system and the supply tank via a conduit. The gas delivery system has a filter to ensure no contaminants can interfere with the safe operation of the system and a pressure sensor to detect if the supply pressure is adequate and thereafter includes a gas shut off valve as a control of the pharmaceutical gas entering the delivery system and to provide safety control in the event the delivery system is over delivering the pharmaceutical gas to the patient. In the event of such over delivery, the shut off valve can be immediately closed and an alarm sounded to alert the user that the gas delivery system has been disabled. As such, the shut off valve can be a solenoid operated valve that is operated from signals directed from a central processing unit including a microprocessor.

A purge valve may be included in the inlet or outlet to purge the system of any other gases that may be in the supply line and refill the supply lines from cylinder to the purge valve with fresh NO/nitrogen so that the system is recharged with the correct supply gas and no extraneous gases, such as ambient air.

In one embodiment of the present invention, the device 10 comprises a first inlet 32 for receiving a therapeutic gas supply comprising nitric oxide; a second inlet 34 for receiving a breathing gas; a therapeutic gas injector module 50 in communication with the therapeutic gas supply to monitor and to control the flow of therapeutic gas to a patient; an outlet in fluid communication with the first inlet 32 and second inlet 34 for supplying breathing gas and therapeutic gas to a patient; a control circuit in communication with the therapeutic gas injector module 50 for triggering an indication or warning when the flow of the breathing gas is outside of a desired range; a computer for determining said one or more respiratory parameters, a ventilator for detecting the level of oxygen ($FiO_2$, Mean Airway Pressure, etc.) in the gas flow passing into or out of the respiratory system of the individual, a proximal pressure transducer 100, a pulse oximeter 90 or other oxygen measurement means for controlling the level of oxygen ($SaO_2$, $SpO_2$, $PaO_2$) in the blood circulation of the individual and producing an output to the computer accordingly, the computer being adapted for calculating, retrieving and storing one or more measurements of oxygenation index .

FIG. 1 illustrates one embodiment of a device 10 for monitoring oxygenation index in accordance with this aspect. First inlet 32 is configured to be placed in fluid communication with a therapeutic gas comprising nitric oxide. Second inlet 34 is configured to be placed in fluid communication with a breathing gas delivery means 30 that provides a breathing gas to a patient, such as a ventilator. Therapeutic injector module 50 is in fluid communication with first inlet 32 and second inlet 34, as well as outlet. Outlet 36 is in fluid communication with first inlet and second inlet, and is configured to supply breathing gas and therapeutic gas to a patient. Flow sensor 40 is in fluid communication and downstream of second inlet 34, and monitors the flow of breathing gas through therapeutic injector module 50. Control circuit 60 is in communication with therapeutic injector module 50, and connects flow sensor to CPU 20. When the flow rate as measured by flow sensor 40 is above or below a predetermined level, central processing unit (CPU) 20 sends a signal to indicator. Indicator can inform a user of the device 10 that the flow is outside of a particular range.

Inspiratory breathing tubing is in fluid communication with outlet 36 and nasal cannula 11. The inspiratory breathing hose 12 provides the gas mixture of breathing gas and therapeutic gas to nasal cannula 11, which delivers the gas mixture to the patient. Patient gas sample line diverts some of the flow of the gas mixture from inspiratory breathing hose and brings it to sample block 120.

Sample block 120, also known as a sample pump, draws some of the flow of the gas mixture through gas sample line . The sample block 120 may be incorporated into the control module 60. The sample block 120 analyzes the concentrations of nitric oxide, oxygen, and nitrogen dioxide in the gas mixture.

The concentrations of nitric oxide, oxygen and nitrogen dioxide measured in the sample block may be shown on display 80.

The flow transducer, also called a flow sensor 40, can be any appropriate flow measuring device. This includes, but is not limited to, a pneumotach, hot wire anemometer, thermal flow sensor, variable orifice, thermal time-of-flight, rotating vane and the like. Also suitable are flow transducers that measure pressure, such as a pressure drop though an orifice, in order to determine flow. According to one embodiment, the flow transducer is part of the therapeutic injector module . In one such embodiment, the flow sensor 40 comprises a hot film sensor and a thermistor. The thermistor measures the temperature of the breathing gas flowing through the injector module. The constant temperature hot film sensor measures the flow of breathing gas, in proportion to the energy required to maintain the platinum film temperature constant. In other embodiments, the flow sensor is upstream of the therapeutic injector module.

The term "control circuit" is intended to encompass a variety of ways that may be utilized to carry out various signal processing functions to operate the therapeutic gas delivery device 10. In a particular embodiment, the control circuit includes a CPU 20 and a flow controller. The CPU 20 can send and receive signals from the flow sensor 40. In a specific embodiment, the CPU 20 may obtain information from the flow sensor 40 and from an input device that allows the user to select the desired dose of nitric oxide.

In a specific embodiment of a control circuit 60, the flow sensor 40 is in communication with a central processing unit (CPU) 20 that monitors the flow of each of the gases to patient as described herein. If a specific dose of nitric oxide is to be administered, the CPU 20 can calculate the necessary flow of therapeutic gas based on the measured flow of breathing gas and the concentration of nitric oxide in the therapeutic gas cylinder.

The central processing unit may be one of any forms of a computer processor that can be used in an industrial or medical setting for controlling various medical gas flow devices and sub-processors. The CPU 20 can be coupled to a memory (not shown) and may be one or more of readily available memory such as random access memory (RAM), read only memory (ROM), flash memory, compact disc, floppy disk, hard disk, or any other form of local or remote digital storage. Support circuits (not shown) can be coupled to the CPU 20 to support the CPU 20 in a conventional manner. These circuits include cache, power supplies, clock circuits, input/output circuitry, subsystems, and the like.

The device 10 also comprises an alert to inform a user of the device 10 when the flow of breathing gas rises above or falls below a predetermined level. In one or more embodiments, the indicator provides an alert when the flow of NO rises above or falls below the predetermined level. In certain embodiments, the alert includes one or more of an audible alert, a visual alert and a text alert. Such alerts can be provided at the location of the device 10 itself, or may be provided at a remote location, such as directly to the medical staff or to a nursing station. When the alert is provided to a remote location, the signal may be transferred from the device 10 to the remote location by any wired or wireless communication. Examples of alerts include text messages, sirens, sounds, alarms, flashing images, changes in display color, or any other means of attracting the attention of a user.

In certain embodiments, the alert includes one or more of an audible alert, a visual alert and a text alert. Such alerts can be provided at the location of the device 10 itself, or may be provided at a remote location, such as directly to the medical staff or to a nursing station.

The device 10 can also include a display 80 that provides a visual and/or numeric indication of the volumetric flow of breathing gas. This visual and/or numeric indication can include any means of displaying the flow of breathing gas, including numerals, graphics, images or the like. The display 80 can also be any sort of appropriate display device, including a dial, gauge or other analog device, or any electronic display device, including an LED, LCD, CRT, etc. Such device need not necessarily be connected to the device 10 and may be utilized in a remote capacity. In certain embodiments, the visual and/or numeric indication includes one or more of volumetric flow rate, tidal volume, and minute ventilation.

The device 10 may comprise an input device that can receive input from a user. Such user input can include operation parameters, such as desired nitric oxide concentration and flow limits. In one embodiment, an input device and display device may be incorporated into one unit, such as a touchscreen device.

The breathing gas delivery system can include any system capable of providing a supply of breathing gas to the patient. The breathing gas may be supplied by ventilatory support, mechanically assisted ventilation or by spontaneous ventilation. Examples of suitable ventilation devices include, but are not limited to, conventional ventilators, jet ventilators, high frequency oscillator ventilators and CPAP device 10. Non-invasive approaches can also be used to supply the breathing gas, including bubble CPAP, SiPAP, nasal cannula and heated high flow nasal cannula.

The therapeutic injector module 50 combines the flow of the breathing gas and the flow of the therapeutic gas. The injector module 50 ensures the proper delivery of inhaled nitric oxide at a set dose based on changes in flow of the breathing gas via communication with the CPU 20. In some embodiments, the therapeutic injector module is a conventional injector module or a neo-injector module.

According to another aspect of the invention, provided is a method of monitoring the delivery of therapeutic gas to a patient comprising: providing a flow of breathing gas; providing a flow of therapeutic gas comprising nitric oxide; delivering the breathing gas and therapeutic gas to a patient; measuring the flow of breathing gas to obtain a measured flow of breathing gas; and displaying the measured flow of breathing gas on a display module.

In specific embodiments, the method further comprises adjusting the flow of breathing gas or NO delivered to the patient in response to the alert. The flow can be adjusted either manually by medical staff, or it may be adjusted automatically by the device 10. According to a certain embodiment, a CPU 20 in communication with the breathing gas delivery means 30 uses clinical decision software to determine when the oxygenation index is below or above the predetermined limit, and sends a signal to the breathing gas delivery means 30 to adjust the breathing gas flow rate to be within the predetermined flow limit.

In one or more embodiments, displaying the measured flow of NO breathing gas includes displaying one or more of volumetric flow rate, tidal volume, and minute ventilation. The displaying can be any visual and/or numeric indication, including numerals, graphics, images or the like. The display module can be performed by any appropriate display device, including a dial, gauge or other analog device, or any electronic display device, including an LED, LCD, CRT, etc.

Once the desired quantity of gaseous drug has been set on the device the system then determines the amount of pharmaceutical gas that is to be delivered in each breath and the amount of time and/or the number of breaths that it will take to deliver the total desired quantity of drug. The monitor display 80 can also display a running total of the delivered dose of NO as it is delivered to the patient, $FiO_2$, Mean Airway Pressure, level of oxygen ($SaO_2$, $SpO_2$, $PaO_2$) and the calculated oxygenation index, so the user can monitor the progress of the treatment. This can be updated each breath as more pharmaceutical gas is delivered.

The device 10 includes a therapeutic gas injector module that is in communication with a control circuit which informs a user when the level of OI, NO, $NO_2$ or flow of a breathing gas rises above a certain level or range or falls below another level or range. Other embodiments pertain to a method of monitoring oxygenation index during the delivery of therapeutic nitric oxide gas to a patient.

In one or more embodiments of the present invention, a device is provided for calculating oxygenation index comprising: a proximal pressure transducer; a $FiO_2$ measurement means; an oxygen measurement means; and a signal processor capable of calculating oxygenation index based upon measurements from these various measurement means. In another embodiment of the present invention, the device may also perform a methemoglobin measurement.

FIG. 1 shows a schematic diagram of an embodiment of the device in accordance with the present invention. As shown in FIG. 1, a supply of nitric oxide is provided in the form of a cylinder of gas 70. The gas is preferably nitric oxide mixed with nitrogen and is a commercially available mixture. Although the preferred embodiment utilizes the present commercial NO/nitrogen mixture, NO may be introduced to the patient via some other gas, preferably an inert gas. Furthermore, a nitric oxide-releasing agent such as nitrogen dioxide ($NO_2$) or a nitrite salt ($NO_2$) may be used with appropriate reducing agents or co-reactants to provide a flow of NO. The pressure sample line may be connected at a sample tee location on the inspiratory limb near the iNO injection point or the gas concentration monitoring point, so that multi-lumen tubes can be used to reduce clutter around the patient. In a preferred embodiment, the pressure sample line is connected as close to the airway as possible to ensure accurate estimation of mean airway pressure. In another embodiment, the pressure transducer may be located inside of a breathing circuit connector close to the patient whereby the measured pressure is transmitted to the CPU by a cable or wireless interface so that a sample line is not needed.

In one more embodiments, iNO gas flow going towards the patient may be momentarily stopped for measurement of mean airway pressure via a pressure sensor placed within the NO delivery device, thereby preventing the need for a separate pneumatic line or transducer placed in the airway. The characterization of pressure drop of the NO delivery tube verses the NO flow delivered would allow for a subtraction of offset to determine mean airway pressure. This method of airway measurement via offset due to NO gas in pressure sense line would be considered a form of purge flow keeping the sense like clean from circuit debris.

A proximal pressure transducer 100 is provided and is preferably attached to a respiratory tube near the patient to measure the pressure in the tube. In one or more embodiments, the proximal pressure transducer 100 measures pressure from a sample line 110 connected to a respiratory tube such as the breathing circuit The proximal pressure transducer data is converted from an analog signal to digital form and processed by a CPU 20 to calculate patient pressure parameters, such as mean airway pressure, peak inhalation pressure and end inhalation pressure (PEEP). Mean airway pressure is the average pressure over the entire respiratory cycle inclusive of background pressure, such as PEEP. The proximal pressure transducer 100 provides a measurement of the patient's circuit pressure as a means of estimating the mean pressure within the respiratory system. It is also utilized for detecting of patient circuit occlusions that may occur. As an alternate to or in addition to a proximal pressure transducer, pressure parameters may be measured by the ventilator or by a respiratory gas monitoring system. Additionally, MAP pressure measurement may be determined through the NO delivery tube and/or within the injector module flow sensor.

In one more embodiments, the device includes a blood oxygenation measurement means, such as a pulse oximeter 90, to measure an oxygen parameter of the patient such as the arterial oxygen saturation ($SaO_2$), peripheral oxygen saturation ($SpO_2$) and/or partial pressure of oxygen in arterial blood ($PaO_2$) (utilizing a continuous blood gas or transcutaneous monitor) of a patient that is using a ventilator. A pulse oximeter relies on the light absorption characteristics of saturated hemoglobin to give an indication of oxygen saturation. The device of various embodiments of the present invention can use a variety of different ventilator systems that are well known in the art. $SaO_2$ indicates the percentage of hemoglobin binding sites in the bloodstream occupied by oxygen and is expressed as a percentage of total hemoglobin. A blood oxygen saturation detector, such as a pulse oximeter, detects information relating to a blood oxygen saturation of a subject which has a close relation to presentation of respiratory failure of the subject. A pulse oximeter is capable of measuring the blood oxygen saturation of a subject patient easily by attaching a probe to a finger tip of the subject. Although a pulse oximeter directly measures peripheral functional oxygen saturation ($SpO_2$) and not arterial oxygen saturation ($SaO_2$), $SpO_2$ is often a good approximation for $SaO_2$ and is less intrusive than a direct measurement of $SaO_2$. However, in some embodiments, $SaO_2$ may be measured directly by drawing a sample of blood and inserting it into a blood gas analyzer.

At low partial pressures of oxygen, most hemoglobin is deoxygenated. At partial oxygen pressures of >10 kPa, around 90% oxygen hemoglobin saturation occurs according to an S curve and approaches 100%. However, several factors can impact the oxygen saturation. The oxygen saturation curve, also known as the oxygen-hemoglobin dissociation curve, is well-known to those skilled in the art and may be used to convert $SaO_2$ values to $PaO_2$ values. Alternatively, the $PaO_2$ may be measured directly by drawing a sample of blood and inserting it into a blood gas analyzer.

The OSI may lose sensitivity as the oxyHb curve flattens out. Accordingly, in one or more embodiments, there may be an upper limit for use.

The oxygen measurement for the patient is measured as an average of the relevant output signal over a predetermined interval of time. The patient's $SaO_2$, $SpO_2$ and $PaO_2$ can vary with each heartbeat and, therefore, an average value is more indicative of the patient's condition at any point in time. Accordingly, an average $SaO_2$, $SpO_2$ or $PaO_2$ value is calculated over an interval of time.

In some embodiments, the $SaO_2$ or $SpO_2$ output signal from a pulse oximeter is averaged over an interval of predetermined time prior to the expiration of the "update time" interval. The CPU 20 averages the measured pulse oximetry of the patient over a predetermined period of time.

The device in FIG. 1 also includes a $FiO_2$ measurement means for obtaining the $FiO_2$ of the breathing gas supplied to the patient. As shown in FIG. 1, the $FiO_2$ may be measured by the ventilator that provides the breathing gas to the patient. However, in other embodiments, the $FiO_2$ is measured by the gas concentration monitoring within the iNO delivery system or may be measured by a respiratory gas monitoring system.

As can be seen from FIG. 1, a flow transducer may also be included which detects the flow of gas from the gas delivery system. As the gas is delivered from the gas delivery system, its flow is sensed by the flow transducer and a signal is transmitted indicative of that flow to the CPU 20. The flow transducer may be of a variety of technologies, including, but not limited to, a pneumotachography, hot wire anemometry, film anemometry, thermal flow sensor, variable orifice, thermal time-of-flight, rotating vane and the like. Also suitable are flow transducers that measure pressure, such as a pressure drop though an orifice, in order to determine flow.

In various embodiments, the device also includes a delivery adapter or therapeutic injector module that combines the flows of breathing gas and therapeutic gas before delivery to the patient. According to one or more embodiments, the flow sensor is part of the therapeutic injector module. In one such embodiment, the flow sensor comprises a hot film sensor and a thermistor. The thermistor measures the temperature of the breathing gas flowing through the injector module. The constant temperature hot film sensor measures the flow of breathing gas, in proportion to the energy required to maintain the platinum film temperature constant. In one or more embodiments, the flow sensor is upstream of the therapeutic injector module.

The flow transducer may be in communication with a control system that monitors the flow of each of the gases to patient as described herein. If a specific dose of nitric oxide is to be administered, a CPU 20 of the control system can calculate the necessary flow of therapeutic gas based on the measured flow of breathing gas and the concentration of nitric oxide or nitric oxide-releasing agent in the therapeutic gas. Such a calculation can be performed using the following equation:

$$Q_{\text{therapeutic}} = [\ \gamma_{\text{set}} / (\gamma_{\text{therapeutic}} - \gamma_{\text{set}})]\ * \ Q_{\text{breathing}}$$

wherein $Q_{\text{breathing}}$ is the flow rate of breathing gas, $\gamma_{\text{set}}$ is the desired nitric oxide concentration, $\gamma_{\text{therapeutic}}$ is the concentration of nitric oxide in the therapeutic gas supply, and $Q_{\text{therapeutic}}$ is the necessary flow of therapeutic gas to provide the desired concentration of nitric oxide in the gas mixture. The necessary $Q_{\text{therapeutic}}$ may then be provided by one or more control valves in communication with the control system.

A signal processing means, such as a CPU 20 is provided to solve certain equations and algorithms to operate the nitric oxide delivery system. In one or more embodiments, the CPU 20 receives a signal from the ventilator, proximal pressure transducer 100 and pulse oximeter 90. The CPU 20 has sufficient information to carry out a calculation of the oxygenation index using the mean airway pressure from the proximal pressure transducer 100, saturation of oxygen from the pulse oximeter 90 and fraction of inspired oxygen from the ventilator. The CPU 20 may contain a microprocessor and associated memory for storing and executing of the programs for calculating oxygenation index, coordination of the ventilator systems, breathing algorithms, alarms, displays and the user interface functions.

In one embodiment, the computer of the device is further adapted for performing a procedure at least once, the procedure comprising calculating oxygenation index based upon a mean airway pressure measurement obtained from a proximal pressure transducer, oxygen measurement obtained from the oxygen measurement means, and $FiO_2$ measurement obtained from the $FiO_2$ measurement means, and retrieving and storing the calculated measurements in the data structure. The collected and calculated data produced thereby may be outputted to a human operator by means of an output device, e.g. a display or monitor, so that the operator can assess the oxygenation index of a patient. Alternatively, the control data item may be used by another part of or a computer program within the computer or by an external control device for automatically control of the means for controlling the flow to the gas-mixing unit of at least one gas.

In one or more embodiments of the present invention, the computer is adapted to determine a parameter relating to an equilibrium state of the overall oxygen uptake or consumption of the individual based on the output of at least one of the proximal pressure transducer, ventilator or pulse oximeter; and to compare said parameter with a predefined threshold value and to produce a control data item accordingly if said parameter exceeds said threshold value.

It is also advantageous if the computer is adapted to assess the appropriate change in oxygen level in the inspired gas ($FiO_2$) from the current oxygen level so as to achieve a given desired target oxygen level in the blood ($SaO_2$) and produce a control data item accordingly so that the oxygen level can be adjusted according to the measured or calculated data. The actual adjustment may be performed by an operator of the device, in which case the calculated or measured data is outputted to an output device.

The assessment of change in oxygen level in the inspired gas may in an embodiment of the invention be based on a predefined set of data representing statistical distributions of variables stored within data storage means associated with the computer and on said measurements. The assessment of change in oxygen level in the inspired gas may be based on the rate of change of the output of at least one of the detection means in response to a change in oxygen level ($FiO_2$) in the inspired gas flow.

The gas delivery unit included in the system can either be a stand-alone device, or any other device, which includes this functionality such as patient ventilation devices. Ventilatory gases are delivered to and removed from the patient/subject through a face mask, mouth piece combined with a nose clip, laryngeal endotracheal tube etc.

Each of the components described above, such as the $FiO_2$ measurement means, proximal pressure transducer or oxygen measurement means, may be all incorporated into the same device, or may be separately added. In some embodiments, a nitric oxide delivery device includes traditional nitric oxide delivery components and one or more of the

FiO$_2$ measurement means, proximal pressure transducer or oxygen measurement means. One exemplary configuration includes a traditional nitric oxide delivery device (such as the INOmax® DSIR delivery system available from INO Therapeutics LLC) that incorporates a proximal pressure transducer and is configured to receive information from a FiO$_2$ measurement means and an oxygen measurement means. Another exemplary configuration includes a traditional nitric oxide delivery device that incorporates a proximal pressure transducer and a FiO$_2$ measurement means (such as an oxygen sensor used to monitor concentration of oxygen administered to the patient) and is configured to receive information from an oxygen measurement means. Yet another exemplary configuration includes a traditional nitric oxide delivery device that incorporates a proximal pressure transducer and is integrated with an oxygen measurement means (such as a pulse oximeter) and is configured to receive information from a FiO$_2$ measurement means such as a ventilator. Another configuration would be to build this functionality into a ventilator or a patient monitoring system.

Calculation of oxygenation index

Oxygenation index is a measure of how well a patient takes in O$_2$ and may be used to assess patient progress with respect to treatment. The Oxygenation Index (OI) is a calculation used in intensive care medicine to measure the fraction of inspired oxygen (FiO$_2$) and its usage within the body. As the oxygenation of a person improves, they will be able to achieve a higher partial pressure of oxygen in arterial blood (PaO$_2$) at a lower FiO$_2$ and/or mean airway pressure (MP AW). The resulting OI will be lower. The OI may be calculated as follows:

Oxygenation Index is often an important parameter in determining whether a patient should begin iNO therapy or Extracorporeal Membrane Oxygenation (ECMO) therapy. For example, a hospital protocol may state that a patient should go on iNO therapy when the OI is > 35.

When calculating the oxygenation index for a patient, it is necessary to determine the mean airway pressure. Currently the mean airway pressure and FiO$_2$ is most commonly measured by the ventilator, which may or may not have proximal pressure sensors.. The mean airway pressure is then calculated by the ventilator over one or more breath cycles continuously. The value is then displayed on the ventilator user interface. To calculate 01, the FiO$_2$ and Mean Airway pressure will be read from the ventilator, and the PaO$_2$ will be read from a pulse oximeter.

The respiratory therapists or pulmonologists may target a mean airway pressure when weaning the patient from the ventilator. Often, adequate oxygenation is a balance between the lowering of mean airway pressure vs FiO$_2$ while trying to find the best combination to prevent lung injury while maintaining adequate blood oxygenation.

Inspired oxygen FiO$_2$ controlled by the ventilator is necessary to improve PaO$_2$ or blood oxygen saturation but can be toxic when provided at higher partial pressures. Oxygen toxicity is a condition resulting from the harmful effects of breathing molecular oxygen (O$_2$) at elevated partial pressures. Managing intensive care ventilation at lower levels of FiO$_2$ is necessary and can be quantified through the reporting of Oxygenation Index.

The device and method of the present invention calculates the oxygenation index of the patient using mean airway pressure measurement obtained from a proximal pressure transducer, oxygen measurement obtained from a pulse oximeter or other oxygen measurement means, and FiO$_2$ measurement obtained from a ventilator or other FiO$_2$ measurement means.

The CPU 20 may calculate the oxygenation index (OI) through the following equation:

$$OI = \frac{F_iO_2 * MAP}{PaO_2}$$

FiO$_2$ = Fraction of inspired oxygen

MAP = Mean airway pressure

PaO$_2$ = Partial pressure of oxygen in arterial blood

The proximal pressure transducer is configured to sense pressure within the breathing circuit of the ventilator. This proximal pressure signal can then be processed through signal processing by the CPU to obtain mean airway pressure. Mean airway pressure is used to calculate oxygenation index along with measurements of fraction of inspired oxygen (FiO$_2$) and PaO$_2$, obtainable from the use of a ventilator and pulse oximeter, respectively. FiO$_2$ is a measure of the level of oxygen coming from ventilator. An oxygen sensor measures the FiO$_2$ delivered by the ventilator. The mean airway pressure obtained from the proximal pressure transducer may be displayed on a monitor.

In one or more embodiments, the device also includes a transmitter to transmit calculated values of methemoglobin, oxygenation index, SpO$_2$, SaO$_2$, and airway pressure to a remote information management system.

The device allows for a continuous monitor of the actual oxygenation index of the patient and therefore may be used as a safety monitor. As a lower oxygenation index indicates a more efficient use of the oxygen supplied by the breathing gases, a lower oxygenation index indicates more successful patient treatment. In the event the oxygenation index rises above a predetermined value established by the user, an alarm may be triggered so the user can attend to the problem.

Accordingly, through the use of the present device, the oxygenation index may be continuously monitored, and compared to a desired predetermined value by the device itself. The system is thus independent and may be readily used with any mechanical ventilator, gas proportioning device or other gas delivery system to deliver a known, desired concentration of NO to a patient.

The device may also provide continuous monitoring of OSI and MetHb, which may be used as a "dosing" monitor for iNO. If the dose is too high, then there will be elevated levels of MetHb. If the dose is too low, the OSI will indicate there is little or no efficacy (unless they are a total non-responder). Alternatively, if the patient is a responder and the OSI is dropping, this would be an indication that the dose of iNO can be lowered and eventually wean the patient off therapy.

The device and method of the present invention may be used to treat or prevent a variety of diseases and disorders, including any disease or disorder that has been treated using any of a gaseous form of nitric oxide, a liquid nitric oxide composition or any medically applicable useful form of nitric oxide. Diseases, disorders, and conditions that may benefit from treatment with, or are associated with, nitric oxide, nitric oxide precursors, analogs, or derivatives thereof, include elevated pulmonary pressures and pulmonary disorders associated with hypoxemia (e.g., low blood oxygen content compared to normal, i.e., a hemoglobin saturation less than 88% and a $PaO_2$ less than 60 mmHg in arterial blood and/or smooth muscle constriction, including pulmonary hypertension, acute respiratory distress syndrome (ARDS), diseases of the bronchial passages such as asthma and cystic fibrosis, other pulmonary conditions including chronic obstructive pulmonary disease, adult respiratory distress syndrome, high-altitude pulmonary edema, chronic bronchitis, sarcoidosis, cor pulmonale, pulmonary embolism, bronchiectasis, emphysema, Pickwickian syndrome, and sleep apnea.

Additional examples of conditions associated with nitric oxide or nitric oxide related treatments include cardiovascular and cardio-pulmonary disorders, such as angina, myocardial infarction, heart failure, hypertension, congenital heart disease, congestive heart failure, valvular heart disease, and cardiac disorders characterized by, e.g., ischemia, pump failure and/or afterload increase in a patient having such disorder, and artherosclerosis. Nitric oxide related treatments may also find use in angioplasty.

Additional examples include blood disorders, including those blood disorders ameliorated by treatment with NO or related molecules, i.e., where NO would change the shape of red blood cells to normal or restore their function to normal or would cause dissolution of blood clots. Examples of blood disorders include, e.g., sickle cell disease and clotting disorders including disseminated intravascular coagulation (DIC), heart attack, stroke, and Coumadin-induced clotting caused by Coumadin blocking protein C and protein S, and platelet aggregation. Additional examples include such conditions as hypotension, restenosis, inflammation, endotoxemia, shock, sepsis, stroke, rhinitis, and cerebral vasoconstriction and vasodilation, such as migraine and non-migraine headache, ischemia, thrombosis, and platelet aggregation, including preservation and processing of platelets for transfusions and perfusion technologies, diseases of the optic musculature, diseases of the gastrointestinal system, such as reflux esophagitis (GERD), spasm, diarrhea, irritable bowel syndrome, and other gastrointestinal motile dysfunctions, depression, neurodegeneration, Alzheimer's disease, dementia, Parkinson's disease, stress and anxiety. Nitric oxide and nitric oxide related treatments may also be useful in suppressing, killing, and inhibiting pathogenic cells, such as tumor cells, cancer cells, or microorganisms, including but not limited to pathogenic bacteria, pathogenic mycobacteria, pathogenic parasites, and pathogenic fungi.

The device may be utilized in the treatment of any patient in which methemoglobinemia or hypoxemia occurs or may occur. These conditions may e.g. be selected from the group comprising left sided heart failure, adult respiratory distress syndrome, pneumonia, postoperative hypoxemia, pulmonary fibrosis, toxic pulmonary lymphoedema, pulmonary embolisms, chronic obstructive pulmonary disease and cardiac shunting.

Reference throughout this specification to "one embodiment," "certain embodiments," "one or more embodiments" or "an embodiment" means that a particular feature, structure, material, or characteristic described in connection with the embodiment is included in at least one embodiment of the invention. Thus, the appearances of the phrases such as "in one or more embodiments," "in certain embodiments," "in one embodiment" or "in an embodiment" in various places throughout this specification are not necessarily referring to the same embodiment of the invention. Furthermore, the particular features, structures, materials, or characteristics may be combined in any suitable manner in one or more embodiments.

Although the invention herein has been described with reference to particular embodiments, it is to be understood that these embodiments are merely illustrative of the principles and applications of the present invention. It will be apparent to those skilled in the art that various modifications and variations can be made to the method and device 10 of the present invention without departing from the spirit and scope of the invention. Thus, it is intended that the present invention include modifications and variations that are within the scope of the appended claims and their equivalents.

The present invention can also be described or defined in accordance with the following clauses:

1. A device for calculating and monitoring oxygenation index during treatment with delivery of nitric oxide comprising:

a first inlet to be placed in fluid communication with a therapeutic gas supply comprising nitric oxide or a nitric oxide-releasing agent;
a second inlet to be placed in fluid communication with a flow of breathing gas;
an outlet to be placed in fluid communication with the first inlet, the second inlet, and a patient;
a proximal pressure transducer for determining mean airway pressure;
a $FiO_2$ measurement means for measuring fraction of inspired oxygen ($FiO_2$);
an oxygen measurement means for measuring one or more oxygen measurements selected from the group consisting of arterial oxygen saturation ($SaO_2$), peripheral oxygen saturation ($SpO_2$) and partial pressure of oxygen in arterial blood (Pa02);
a signal processor capable of calculating oxygenation index based upon a mean airway pressure measurement obtained from the proximal pressure transducer, an oxygen
measurement obtained from the oxygen measurement means, and a $FiO_2$ measurement obtained from the $FiO_2$ measurement means; and
a display.

2. The device of clause 1 wherein said signal processor is a central processing unit.
3. The device of clause 1 or 2, wherein the $FiO_2$ measurement means comprises a ventilator.
4. The device of any of clauses 1-3, wherein the $FiO_2$ measurement means comprises a $FiO_2$ sensor.
5. The device of any of clauses 1-4, wherein the oxygen measurement means comprises a pulse oximeter and the oxygen measurement comprises $SpO_2$.
6. The device of any of clauses 1-5, further comprising a blood gas monitor to measure $SpO_2$.
7. The device of any of clauses 1-6, further including an alarm system operable by a signal from said signal processor indicative of a predetermined value of oxygenation index, $SpO_2$,
$SaO_2$, methemoglobin, or airway pressure.
8. The device of any of clauses 1-7, further comprising a flow transducer to measure the flow of breathing gas and a control system in communication with the flow transducer.
9. The device of any of clauses 1-8, further comprising one or more control valves to deliver a flow of the therapeutic gas comprising nitric oxide or a nitric oxide-releasing agent in an amount to provide a predetermined concentration of nitric oxide to a patient.
10. The device of any of clauses 1-9, wherein the flow transducer is integral to an injector module that combines the flows of breathing gas and therapeutic gas comprising nitric oxide or a nitric oxide-releasing agent.
11. The device of any of clauses 1-10, wherein the signal processor is configured to communicate with the $FiO_2$ measurement means that measures fraction of inspired oxygen ($FiO_2$) and an oxygen measurement means that measures one or more oxygen measurements selected from the group consisting of arterial oxygen saturation ($SaO_2$), peripheral oxygen saturation ($SpO_2$) and partial pressure of oxygen in arterial blood ($PaC_2$).
12. The device of any of clauses 1-11, wherein the display shows calculated values of methemoglobin, oxygenation index, $SpO_2$, $SaO_2$, and airway pressure.
13. The device of any of clauses 1-12, further comprising a transmitter to transmit calculated values of methemoglobin, oxygenation index, $SpO_2$, $SaO_2$, and airway pressure to a remote information management system.
14. The device of any of clauses 1-13, further comprising a purge valve
15. A method of monitoring oxygenation index comprising the steps of: (a) obtaining a mean airway pressure (MAP) measurement from a proximal pressure transducer;

(b) obtaining one or more oxygen measurements selected from the group consisting of arterial oxygen saturation ($SaO_2$), peripheral oxygen saturation ($SpO_2$) and partial pressure of oxygen in arterial blood ($PaO_2$) from an oxygen measurement means;
(c) obtaining a fraction of inspired oxygen ($FiO_2$) measurement from a $FiO_2$ measurement means;
(d) transmitting the MAP measurement, the oxygen measurement and the $FiO_2$ measurement to a signal processor;
(e) calculating an oxygenation index (OI) value via the signal processor using the following equation

$$OI = \frac{F_iO_2 * MAP}{PaO_2}$$

(f) conveying the oxygenation index to an end user via a display.

16. The method of clause 15, further comprising administering a therapeutic gas comprising nitric oxide to a patient.

17. The method of clause 15 or 16, further comprising comparing the oxygenation index value to a predetermined high value limit and emitting an alarm if the oxygenation index is above the high value limit.

**Claims**

1. A method for calculating and monitoring an oxygenation index, the method comprising:

supplying to a first inlet a flow of nitric oxide from a therapeutic gas supply comprising nitric oxide or a nitric oxide-releasing agent;

supplying to a second inlet a flow of a breathing gas from a breathing gas delivery means;

combining the flow of nitric oxide and the flow of the breathing gas in an injector module to produce a combined flow of nitric oxide and the breathing gas;

providing the combined flow of nitric oxide and the breathing gas to a breathing hose;

measuring a mean airway pressure (MAP) of the combined flow of the therapeutic gas and the breathing gas utilizing a proximal pressure transducer located along a sample line;

measuring a fraction of inspired oxygen ($FiO_2$) measurement utilizing a $FiO_2$ measurement means;

measuring, utilizing an oxygen measurement means, one or more oxygen measurements selected from arterial oxygen saturation ($SaO_2$), peripheral oxygen saturation ($SpO_2$), partial pressure of oxygen in arterial blood ($PaO_2$), and/or methemoglobin;

measuring the flow of the breathing gas utilizing a flow transducer that is integral to the injector module;

calculating, utilizing a signal processor, the oxygenation index based upon the mean airway pressure measurement, the oxygen measurements obtained, and the $FiO_2$ measurement means;

presenting on a display a representation of the oxygenation index;

calculating if the oxygenation index is below or above a predetermined limit; and

automatically adjusting the flow of the breathing gas from the breathing gas delivery means such that the oxygenation index is within the predetermined limit.

2. The method of claim 1 wherein the one or more oxygen measurements include at least one of arterial oxygen saturation ($SaO_2$) and peripheral oxygen saturation ($SpO_2$).

3. The method of claim 1, wherein the $FiO_2$ measurement means comprises a $FiO_2$ sensor.

4. The method of claim 1, wherein the oxygen measurement means comprises a pulse oximeter and the oxygen measurement comprises $SpO_2$.

5. The method of claim 1, further comprising the step of monitoring $SpO_2$ utilizing a blood gas monitor.

6. The method of claim 1, further comprising the step of providing an alarm when a signal from the signal processor indicates one or more of a predetermined value of oxygenation index, $SpO_2$, $SaO_2$, and/or mean airway pressure.

7. The method of claim 1, wherein the step of supplying the therapeutic gas includes supplying nitric oxide in an amount to provide a predetermined concentration of nitric oxide by operating one or more control valves.

8. The method of claim 1, wherein the signal processor is configured to communicate with the $FiO_2$ measurement means and the oxygen measurement means.

9. The method of claim 1, wherein the step of presenting on the display includes presenting calculated values of methemoglobin, the oxygenation index, $SpO_2$, $SaO_2$, and the mean airway pressure.

10. The method of claim 1, further comprising the step of transmitting calculated values of methemoglobin, the oxygenation index, $SpO_2$, $SaO_2$, and the mean airway pressure to a remote information management system.

11. The method of claim 1, further comprising the step of purging a supply line using a purge valve to remove an extraneous gas, wherein the purge valve is connected to the first inlet.

12. A method of monitoring oxygenation index comprising the steps of: (a) obtaining a mean airway pressure (MAP) measurement from a proximal pressure transducer;

   (b) obtaining one or more oxygen measurements selected from the group consisting of arterial oxygen saturation ($SaO_2$), peripheral oxygen saturation ($SpO_2$) and partial pressure of oxygen in arterial blood ($PaO_2$) from an oxygen measurement means;
   (c) obtaining a fraction of inspired oxygen ($FiO_2$) measurement from a $FiO_2$ measurement means;
   (d) transmitting the MAP measurement, the oxygen measurement and the $FiO_2$ measurement to a signal processor;
   (e) calculating an oxygenation index (OI) value via the signal processor using the following equation

$$OI = \frac{F_iO_2 * MAP}{PaO_2} \, ;$$

   andp
   (f) conveying the oxygenation index to an end user via a display.

13. The method of claim 12, further comprising administering a therapeutic gas comprising nitric oxide to a patient.

14. The method of claim 12, further comprising comparing the oxygenation index value to a predetermined high value limit and emitting an alarm if the oxygenation index is above the high value limit.

Figure 1

EP 3 653 119 A1

EP 3 653 119 A1

Europäisches
Patentamt

European
Patent Office

Office européen
des brevets

# EUROPEAN SEARCH REPORT

Application Number

EP 19 20 6524

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DELLINGER R PHILLIP ET AL: "Effects of inhaled nitric oxide in patients with acute respiratory distress syndrome: Results of a randomized phase II trial", CRITICAL CARE MEDICINE, LIPPINCOTT WILLIAMS & WILKINS, US, vol. 26, no. 1, 1 January 1998 (1998-01-01), pages 15-23, XP009131994, ISSN: 0090-3493 * the whole document * | 1-11 | INV.<br>A61B5/08<br>A61B5/1455<br>A61M16/12 |
| X | US 2004/118407 A1 (KANDLER MICHAEL [DE]) 24 June 2004 (2004-06-24) * paragraphs [0066], [0067]; figure 1 * | 12-14 | |
| A | US 6 597 939 B1 (LAMPOTANG SAMSUN [US] ET AL) 22 July 2003 (2003-07-22) | 1-14 | |
| A | US 2009/241957 A1 (BAKER JR CLARK R [US]) 1 October 2009 (2009-10-01) * the whole document * | 1-14 | |
| A | WO 2012/080903 A1 (KONINKL PHILIPS ELECTRONICS NV [NL]; GARDE SMITA [US]; AHMAD SAMIR [US] 21 June 2012 (2012-06-21) * paragraph [0018]; figure 1 * | 1-14 | TECHNICAL FIELDS SEARCHED (IPC)<br><br>A61B<br>A61M |
| A | US 2011/041849 A1 (CHEN BO [US] ET AL) 24 February 2011 (2011-02-24) * the whole document * | 1-14 | |
| A | US 2006/266355 A1 (MISHOLI BOAZ [US]) 30 November 2006 (2006-11-30) * paragraph [0006] * | 1-14 | |

The present search report has been drawn up for all claims

| Place of search | Date of completion of the search | Examiner |
|---|---|---|
| Munich | 30 March 2020 | Schindler, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

15

## ANNEX TO THE EUROPEAN SEARCH REPORT
## ON EUROPEAN PATENT APPLICATION NO.

EP 19 20 6524

This annex lists the patent family members relating to the patent documents cited in the above-mentioned European search report.
The members are as contained in the European Patent Office EDP file on
The European Patent Office is in no way liable for these particulars which are merely given for the purpose of information.

30-03-2020

| Patent document cited in search report | | | Publication date | Patent family member(s) | | | Publication date |
|---|---|---|---|---|---|---|---|
| US 2004118407 | A1 | | 24-06-2004 | EP | 1379306 | A1 | 14-01-2004 |
| | | | | JP | 2004524933 | A | 19-08-2004 |
| | | | | PL | 363283 | A1 | 15-11-2004 |
| | | | | US | 2004118407 | A1 | 24-06-2004 |
| | | | | WO | 02083222 | A1 | 24-10-2002 |
| US 6597939 | B1 | | 22-07-2003 | AU | 754605 | B2 | 21-11-2002 |
| | | | | CA | 2323454 | A1 | 26-08-1999 |
| | | | | EP | 1056393 | A2 | 06-12-2000 |
| | | | | JP | 2002503507 | A | 05-02-2002 |
| | | | | US | 6597939 | B1 | 22-07-2003 |
| | | | | US | 2001014772 | A1 | 16-08-2001 |
| | | | | WO | 9942034 | A2 | 26-08-1999 |
| US 2009241957 | A1 | | 01-10-2009 | EP | 2257328 | A2 | 08-12-2010 |
| | | | | US | 2009241957 | A1 | 01-10-2009 |
| | | | | US | 2009241958 | A1 | 01-10-2009 |
| | | | | WO | 2009120639 | A2 | 01-10-2009 |
| WO 2012080903 | A1 | | 21-06-2012 | BR | 112013014920 | A2 | 13-09-2016 |
| | | | | CN | 103379934 | A | 30-10-2013 |
| | | | | EP | 2651478 | A1 | 23-10-2013 |
| | | | | JP | 6235342 | B2 | 22-11-2017 |
| | | | | JP | 2014501130 | A | 20-01-2014 |
| | | | | RU | 2013132964 | A | 27-01-2015 |
| | | | | US | 2013312754 | A1 | 28-11-2013 |
| | | | | WO | 2012080903 | A1 | 21-06-2012 |
| US 2011041849 | A1 | | 24-02-2011 | EP | 2467185 | A1 | 27-06-2012 |
| | | | | US | 2011041849 | A1 | 24-02-2011 |
| | | | | US | 2014083426 | A1 | 27-03-2014 |
| | | | | WO | 2011022242 | A1 | 24-02-2011 |
| US 2006266355 | A1 | | 30-11-2006 | AT | 461722 | T | 15-04-2010 |
| | | | | BR | PI0609985 | A2 | 18-10-2011 |
| | | | | CA | 2609856 | A1 | 30-11-2006 |
| | | | | EP | 1893269 | A2 | 05-03-2008 |
| | | | | JP | 5016595 | B2 | 05-09-2012 |
| | | | | JP | 2008541849 | A | 27-11-2008 |
| | | | | US | 2006266355 | A1 | 30-11-2006 |
| | | | | WO | 2006127356 | A2 | 30-11-2006 |

EPO FORM P0459

For more details about this annex : see Official Journal of the European Patent Office, No. 12/82